# EUROPÄISCHE PATENTSCHRIFT

(11) **EP 3 230 928 B1**
(45) Veröffentlichungstag und Bekanntmachung des Hinweises auf die Patenterteilung: **01.04.2020**
(21) Anmeldenummer: 15817088.6
(22) Anmeldetag: 01.12.2015
(51) Int. Cl.: G06M 1/14, G06M 1/16, G06M 1/04

(54) **ZÄHLWERK**
COUNTER
COMPTEUR

(30) Priorität: 10.12.2014 DE 102014118325
(43) Veröffentlichungstag der Anmeldung: 18.10.2017
(73) Patentinhaber: Von Schuckmann, Alfred, 47627 Kevelaer (DE)
(72) Erfinder: Von Schuckmann, Alfred, 47627 Kevelaer (DE)
(74) Vertreter: Müller, Enno
(86) Internationale Anmeldenummer: PCT/EP2015/078170
(87) Internationale Veröffentlichungsnummer: WO 2016/091652

(56) Entgegenhaltungen:
- WO-A1-92/09324
- WO-A1-2008/121459
- WO-A2-99/36115

## Beschreibung

Die Erfindung betrifft zunächst ein Handgerät zur Ausgabe einer pharmazeutischen Substanz, mit einem Gehäuse und einem Zählwerk, nach den Merkmalen des Oberbegriffs des Anspruches 1.

Die Erfindung betrifft des Weiteren ein Zählwerk für ein Handgerät zur Ausgabe einer pharmazeutischen Substanz nach den Merkmalen des Oberbegriffs des Anspruches Bevorzugte Ausführungsformen sind in den abhängigen Ansprüchen 3 bis 10 definiert.

Derartige Zählwerke sind bereits in verschiedener Hinsicht bekannt geworden. Insbesondere ist auf die WO 2007/104694 A1 (US 8 132 565 B2) zu verweisen. Hieraus ist ein Zählwerk bekannt, das im konkreten Benutzungsfall oberseitig eines Vorratsraumes des Handgerätes, welcher Vorratsraum die pharmazeutische Substanz aufnimmt, angeordnet ist. Es wird zufolge einer unmittelbaren Druckbetätigung durch den Benutzer beaufschlagt. Das Antriebsteil ist mit einer Drehachse in zwei Lagerungsausformungen aufgenommen, die innenseitig einer Betätigungstaste nach unten ragend angeformt sind. Das Zählrad ist zwischen der Betätigungstaste und einer Gehäusewandung, welche beide Teile jeweils auch Teile des Gehäuses sind, aufgenommen.

Weiter ist zum Stand der Technik auf die WO 2006/ 051073 A1 (US 7 448 342 B2) zu verweisen. Das hieraus bekannte Zählwerk ist im Benutzungszustand unterhalb eines geschlossenen Behältnisses vorgesehen, welches die pharmazeutische Substanz aufnimmt und in dem Handgerät angeordnet ist. Es wird durch das Niederdrücken des Behältnisses betätigt. Das Zählrad ist zwischen einem unteren Ringteil und einer oberen Gehäuseabdeckung aufgenommen. Das Antriebsteil ist lose eingelegt und wirkt über ein Scheibenteil, weiter auch vermittels eines Planetenrades - erst - auf das Zählrad ein. Beim Gegenstand der zunächst genannten Druckschrift ist weiter als Rückstellfeder lediglich die die Betätigungstaste insgesamt zurückstellende, zwischen der Betätigungstaste und einem Gehäuse-Zwischenboden aufgenommene Rückstellfeder vorgesehen. Beim Gegenstand der weiter genannten Druckschriften weist das Antriebsteil in sich federbare Antriebsfinger auf, die auf das Scheibenteil einwirken.

Aus der WO 2008/121459 A1 ist ein Zählwerk bekannt, bei welchem das Antriebsteil drehbeweglich relativ zu dem Halterungsteil ausgebildet ist. Der untere Rand des Zählrades ist geschlossen als umlaufende Randkante ausgebildet. Die Rückstellfeder wirkt mit einem Zwischenrad zusammen. Sie ist in einer Beaufschlagungsrichtung oberhalb des Halterungsteils angeordnet.

Aus der WO 92/09324 A1 ist ein Zählwerk bekannt, bei welchem das Zählrad am oberen Rand eine Verzahnung aufweist und das Antriebsteil vermittels einer Halterung an dem Halterungsteil angebracht ist. Das Zählwerk als solches ist nicht durch eine Rückstellfeder rückstellbar. Die nur auf das Antriebselement wirkende Rückstellfeder ist nicht zum Antrieb genutzt. Die Drehachse des Antriebsteils verläuft außerhalb eines Teiles, das als Zählrad angesprochen ist.

Aus der WO 99/36115 A2 ist ein Zählwerk bekannt, das scheibenförmig ausgebildet ist. Außerhalb der Randkante des Zählrades ist ein gesondertes Verzahnungsteil vorgesehen.

Ausgehend von dem dargelegten Stand der Technik beschäftigt sich die Erfindung mit der Aufgabenstellung, ein vorteilhaftes Handgerät mit einem Gehäuse und einem Zählwerk sowie ein vorteilhaft ausgebildetes Zählwerk anzugeben.

Diese Aufgabe ist hinsichtlich des Handgerätes mit einem Gehäuse und einem Zählwerk beim Gegenstand des Anspruchs 1 gelöst, wobei darauf abgestellt ist, dass das Zählrad zylindrisch ausgebildet ist und mit einer an einer im Benutzungszustand unteren Randkante ausgebildeten Eingriffsverzahnung versehen ist, auf welche das Antriebsteil zur Drehung auf das Zählrad einwirkt, wobei eine nach oben verbleibende Außenfläche des Zählrades zur Anbringung von lesbaren Zeichen frei nutzbar ist, und dass zur Durchführung einer Zählung das Halterungsteil zusammen mit dem Antriebsteil zu dem relativ zu dem Gehäuse praktisch feststehenden, allenfalls etwa im Sinne einer Bewegung quer zur Einfederungsbewegung bei einer Betätigung bewegten Eingriffsteil bewegt ist.

Die genannte Aufgabe ist weiter hinsichtlich des Zählwerkes für ein Handgerät beim Gegenstand des Anspruches 2 gelöst, wobei darauf abgestellt ist, dass die in einer Beaufschlagungsrichtung unterhalb des Halterungsteils angeordnete Rückstellfeder in Form eines Bügels gebildet ist, der zwei Endbereiche aufweist, die an dem Halterungsteil befestigt sind, wobei die Endbereiche an unterschiedlichen Stellen unterseitig des Halterungsteils in dieses übergehen und dass zur Durchführung einer Zählung das Halterungsteil zusammen mit dem Antriebsteil relativ zu dem Eingriffsteil bewegbar ist.

Das Halterungsteil ist hinsichtlich des Handgerätes unabhängig von dem Gehäuse ausgebildet und an dem Halterungsteil auch das Antriebsteil angebracht. Dies unmittelbar, ohne ein vermittelndes Teil zu dem Halterungsteil. Das Antriebsteil kann hierzu über einen Formschluss, bspw. eine Verrastung, mit dem Halterungsteil verbunden sein. Dadurch, dass an dem Halterungsteil auch das Antriebsteil angebracht ist, ist eine sehr einfache Ausgestaltung gegeben. Eines weiteren Teiles bedarf es nicht. Das Halterungsteil bildet nicht zugleich einen Teil des Gehäuses des Handgerätes.

Insbesondere nicht zugleich eine Außenwandung des Gehäuses. Vielmehr ist das Zählwerk mit dem Halterungsteil zum Einsetzen in ein Gehäuse des Handgerätes ausgebildet.

Die Rückstellfeder, die in Beaufschlagungsrichtung des Zählwerkes unterhalb des Zählwerkes, damit insbesondere unterhalb des Halterungsteils und des Zählrades, angeordnet ist, kann eine weitere Funktion, nämlich eine unmittelbare Zusammenwirkung mit dem Antriebsteil, um den Antrieb und damit die Drehung des Zählrades zu bewirken, aufweisen. Auch hierdurch lässt sich günstig ein Teil einsparen und entsprechend auch eine kompakte Gestaltung des Zählwerks erreichen. Das Eingriffsteil wird im Zuge einer Auslenkung der Rückstellfeder aus einer Ruhestellung in eine Betätigungsstellung mit dem Antriebsteil in Eingriff gebracht. In der Ruhestellung liegen das Eingriffsteil und das Antriebsteil bevorzugt nicht aneinander an. Im Zuge einer Betätigung bewegt sich das Eingriffsteil, einschließlich auch eines tatsächlich in Eingriff stehenden Eingriffsendes, relativ, und zwar in Richtung einer Mittelachse des Zählrades und/oder des Halterungsteils, relativ zu dem Halterungsteil und dem Zählrad.

Das zylindrisch ausgebildete Zählrad kann außen, zugeordnet der Fläche, auf der gewöhnlich die lesbaren Zeichen angebracht sind, das Antriebsteil aufweisen. Hierdurch ist das Zählrad auf einer Seite gleichsam vorbauend mit dem Antriebsteil versehen. Andererseits kann das Zählrad auf der anderen Seite unmittelbar einem Fenster oder einem durchsichtigen Teil des Handgerätes, in dem es anzubringen ist, zugeordnet sein. Bei einem Antriebsteil, dessen Drehachse außerhalb des Zählrades verläuft, lässt sich von außen eine günstige Einwirkung auf das Zählrad erreichen. Es kann bei einer zylindrischen oder kreisringförmigen oder jedenfalls gekrümmten Ausbildung des Zählrades die konvexe (Außen-) Seite des Zählrades genutzt werden.

Es ist weiter bevorzugt, dass das Eingriffsteil an dem Halterungsteil angebracht ist. Das Halterungsteil haltert dann bevorzugt nicht nur das Antriebsteil, sondern auch das mit dem Antriebsteil zusammenwirkende Eingriffsteil.

Insbesondere kann das Eingriffsteil an dem Halterungsteil vermittels einer beziehungsweise der genannten Rückstellfeder angebracht sein. Das Eingriffsteil kann mit der Rückstellfeder einteilig, insbesondere materialeinheitlich einteilig ausgebildet sein.

Bevorzugt ist jedenfalls auch das Halterungsteil materialeinheitlich einteilig mit einer Halterungsausformung für das Antriebsteil ausgebildet.

Zur Durchführung einer Zählung ist das Halterungsteil zusammen mit dem Antriebsteil zu dem bei dem Handgerät relativ zu dem Gehäuse feststehenden Eingriffsteil bewegbar. Wenn das Eingriffsteil, wie bevorzugt, verbunden mit der Rückstellfeder ausgebildet ist, kann sich zwar zufolge der Einfederungsbewegung auch eine gewisse Bewegung der Rückstellfeder quer zu der Einfederungsbewegung ergeben. Das Eingriffsteil ist jedoch bevorzugt mit dem Bereich der Rückstellfeder verbunden beziehungsweise einteilig ausgebildet, der auch im entspannten Zustand in Anlage an dem Gehäuse, insbesondere einem Boden des Gehäuses des Handgerätes ist. Insofern bewegt sich dieser Bereich praktisch nicht, zumindest nicht vertikal beziehungsweise in Einfederungsrichtung der Rückstellfeder, sondern allenfalls etwas im Sinne einer Bewegung quer zur Einfederungsbewegung bei einer Betätigung.

Weiter kann an dem Halterungsteil ein Sperrteil zur Einwirkung auf das Antriebsteil angebracht sein. Insbesondere ist auch bevorzugt, dass das Sperrteil einteilig, weiter bevorzugt materialeinheitlich einteilig bereits mit dem Halterungsteil ausgeführt ist. Somit hat das Halterungsteil noch eine weitere Funktion übernommen, was weiter dazu beiträgt, ein Zählwerk mit möglichst wenigen Einzelteilen zur Verfügung zu stellen.

Das Zählrad kann, wie schon angesprochen, zylindrisch ausgebildet sein, mit einer bevorzugt an einer im Benutzungszustand unteren Randkante ausgebildeten Eingriffsverzahnung. Dadurch, dass die Eingriffsverzahnung bevorzugt an der unteren Randkante des weiter bevorzugt zylindrischen Zählrades ausgebildet ist, kann die verbleibende (Außen-)Fläche (nach oben) zur Aufbringung der lesbaren Zeichen frei genutzt werden.

Die zylindrische Ausbildung des Zählrades, ohne dass hierin, im Inneren des so gebildeten Zylinderkörpers, auch weitere Einbauteile vorgesehen sind, ist auch günstig zur Zusammenwirkung mit einem Vorratsbehältnis des Handgerätes, in welchem die pharmazeutische Substanz aufgenommen ist. Im Inneren des Zählrades befinden sich bevorzugt nur Abschnitte des Halterungsteils. Das Vorratsbehältnis ist typischerweise als sogenannter Kanister ausgebildet, der eine im Längsquerschnitt untere stufenförmige Gestaltung, mit einem mittigen zapfenartigen Vorsprung aufweist, aus welchem Vorsprung dann letztlich ein rohrförmiges Ventilteil herausragt, aus dem die Substanz bei einer Betätigung ausgebracht wird.

Die zylindrische Ausgestaltung des Zählrades kann insbesondere so vorgesehen sein, dass eine obere Stirnfläche des Zylinderkörpers der unteren äußeren Stufenfläche des genannten Vorratsbehältnisses zugeordnet angeordnet sein kann. Das Vorratsbehältnis ist typischerweise gegen eine in dem Vorratsbehältnis selbst angebrachte Feder zur Betätigung von einem Benutzer nach unten zu drücken, womit dann gleichzeitig zufolge der Druckausübung auf das Zählrad beziehungsweise das das Zählrad aufnehmende Halterungsteil die Bewegung erfolgt, welche letztlich zur Betätigung des Zählwerkes führt.

Das Halterungsteil ist bevorzugt in gleicher Weise zylindrisch ausgebildet.

Bevorzugt ist auch, dass das Halterungsteil das Zählrad in einem Längsquerschnitt von innen übergreift. Somit liegt das Zählrad außenseitig mit seiner Außenfläche frei. Diese Außenfläche trägt bevorzugt die schon angesprochenen lösbaren Zeichen.

Das Antriebsteil kann bevorzugt in weiterer Einzelheit als Schneckenwelle mit Verzahnungsvorsprüngen ausgebildet sein. Dies bedeutet, dass einerseits eine Umfangsverzahnung vorgesehen ist, diese Umfangsverzahnung an dem Antriebsteil jedoch an einem zu einer Drehachse des Antriebsteils schräg verlaufenden, sich schneckenartig um die Drehachse erstreckenden Wendelgang ausgebildet ist.

Das Antriebsteil ist bevorzugt derart angeordnet, dass seine Drehachse quer zu einer Mittelachse des Zählrades und/oder des Halterungsteiles, die bevorzugt eine Zylinderachse ist, bei entsprechender Ausbildung des Zählrades und/oder des Halterungsteils, verläuft.

Das Eingriffsteil wirkt bevorzugt zählradseitig der Drehachse des Antriebsteiles auf das Antriebsteil ein. Somit ist ein sehr naher Angriffspunkt zu der Kraftüberleitung von dem Antriebsteil auf das Zählrad erreicht. Die beschriebenen Verzahnungsvorsprünge dienen auch gerade der Zusammenwirkung zwischen dem Eingriffsteil und dem Antriebsteil sowie auch dem Sperrteil.

Das Sperrteil wirkt bevorzugt auf einer dem Zählrad abgewandten Seite der Drehachse des Antriebsteils auf dieses ein. Somit ist zunächst eine gegenüberliegende Einwirkung - bezogen auf die Drehachse des Sperrteils und des Antriebsteils - erreicht. Zugleich können die genannten Teile gegeneinander fahren, ohne dass die Gefahr einer Behinderung gegeben ist.

Das Sperrteil und das Eingriffsteil sind bevorzugt gegensinnig zueinander gerichtet angeordnet. Die freien Enden des Sperrteils und des Eingriffsteils sind zueinander gerichtet angeordnet. Hierbei kann im Einzelnen - bezogen auf einen beschriebenen Einbauzustand, bei welchem das Zählwerk von oben von dem Vorratsbehältnis beaufschlagt ist - das Sperrteil oberseitig und das Eingriffsteil unterseitig angeordnet sein.

Das Eingriffsteil und/oder das Sperrteil sind - bezogen auf eine Ansicht von oben oder unten, in welcher sich eine Mittelachse des Zählrades und/oder des Halterungsteils punktförmig abbildet - außerhalb zu dem Zählrad beziehungsweise dem Halterungsteil im Übrigen angeordnet. Die Anordnung außerhalb des Halterungsteils im Übrigen bezieht sich darauf, dass die Anbindung für das Eingriffsteil, die - bezogen auf eine Ausführungsform - durch die Rückstellfeder selbst gegeben sein kann beziehungsweise die Anbindung für das Sperrteil, die insoweit dann auch Teile des Halterungsteils sind, bereits, soweit sie in der genannten Ansicht sichtbar sind, als außerhalb des Halterungsteils liegend angesehen werden.

Die Rückstellfeder kann auch noch in der genannten Ansicht über das Eingriffsteil und/oder das Sperrteil seitlich hinausreichen.

Die Rückstellfeder selbst ist bevorzugt in Form eines Bügels, insbesondere eines geschlossenen Bügels, gebildet. Der Bügel weist zwei Enden auf, die an dem Halterungsteil befestigt sind. Die Befestigungsbereiche, beispielsweise in Form von Anbindungspunkten, können sich hinsichtlich einer Durchmesserlinie des bevorzugt zylindrisch gebildeten Halterungsteils, etwa bezogen auf den durch die Halterungswände gegebenen Zylinder, gegenüberliegen. Sie können in Bezug eines hierdurch gebildeten Kreises des Zylinders auch auf einer Sekante sich gegenüberliegen. In letzterem Fall sind die Befestigungsbereiche beziehungsweise die Anbindungspunkte bevorzugt derart versetzt zu der Mittelachse angeordnet, dass die Mittelachse zwischen der Sekante und dem Sperrteil und/oder dem Eingriffsteil liegt, dieses wiederum bezogen auf die Ansicht, beispielsweise von oben, in welcher sich die Mittelachse punktförmig abbildet.

Das Zählrad ist bevorzugt als einteiliges Zylinderteil gebildet.

Nachstehend ist die Erfindung weiter anhand der beigefügten Zeichnung erläutert, die aber lediglich ein Ausführungsbeispiel darstellt. Hierbei zeigt:
- Fig. 1: eine beispielsweise perspektivische Außenansicht eines Handgerätes mit darin angeordnetem Vorratsbehältnis und (nicht sichtbarem) Zählwerk;
- Fig. 2: einen Querschnitt durch den Gegenstand der Figur 1, geschnitten entlang der Ebene II-II. Hierbei ist schematisch die Anordnung des Zählwerks in dem Gehäuse des Handgerätes dargestellt;
- Fig. 3: eine Herausvergrößerung des Bereichs III-III in Fig. 2;
- Fig. 4: eine Explosionsdarstellung des Zählwerks, in der Version mit an dem Gehäuse angeformten Eingriffsteil;
- Fig. 5: eine Explosionsdarstellung gemäß Fig. 4, mit an der Rückstellfeder angeformtem Eingriffsteil
- Fig. 6: einen Querschnitt durch das auf das Halterungsteil aufgesetzte Zählrad;
- Fig. 7: eine schematische Draufsicht auf das Gehäuse des Handgerätes bei entnommenen Zählwerk, gesehen auf den Boden des Gehäuses, in der Version mit an dem Gehäuse angeformtem Eingriffsteil; und
- Fig. 8: eine Darstellung gemäß Fig. 7, in der Version mit an der Rückstellfeder angeformtem Eingriffsteil.

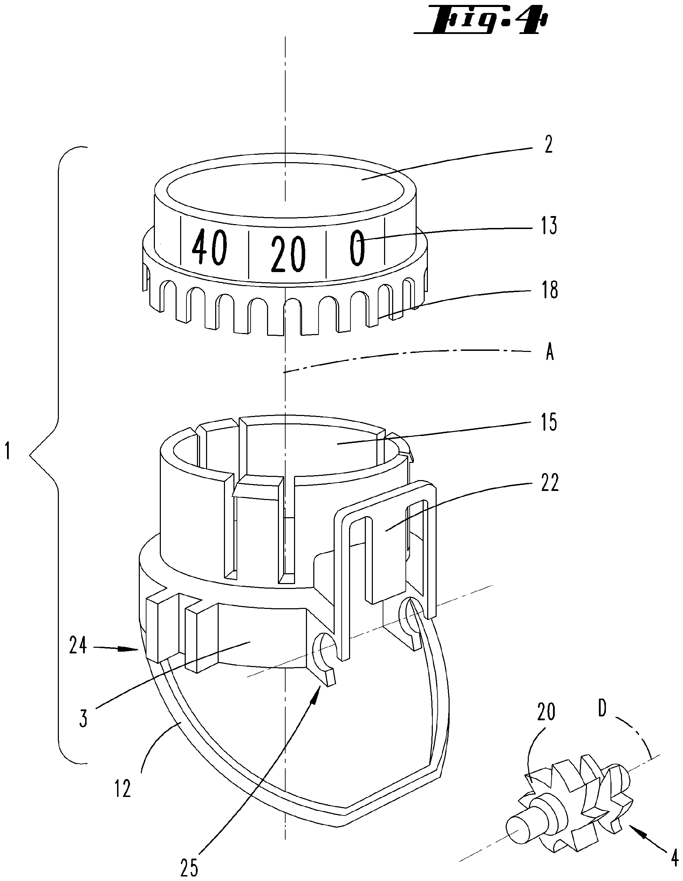

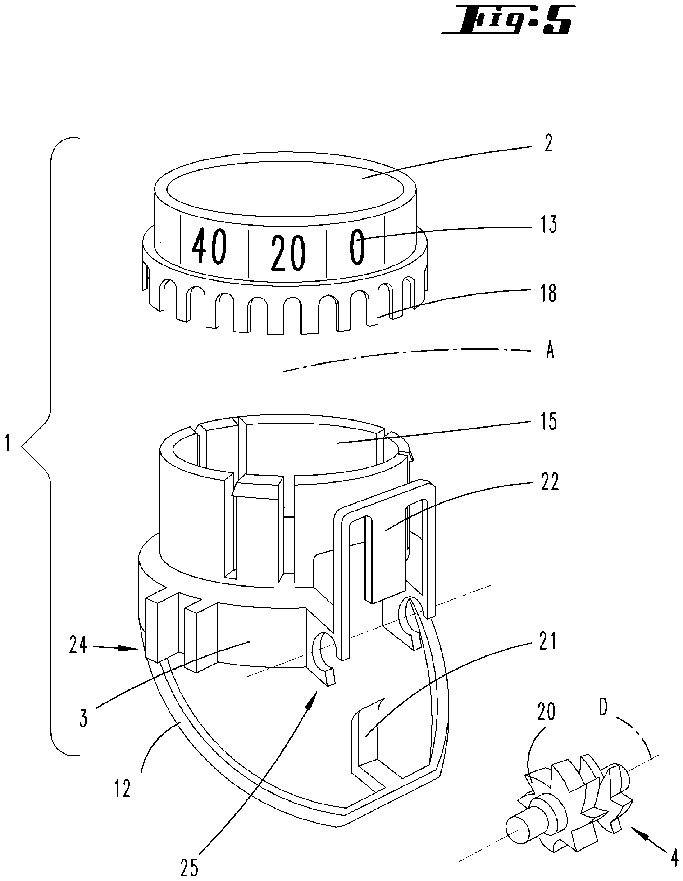

Dargestellt und beschrieben ist, vergleiche insbesondere Figuren 4, 5, ein Zählwerk 1, das aus einem Zählrad 2, einem Halterungsteil 3 und einem Antriebsteil 4 besteht.

Schon diese wenigen Teile, ersichtlich bevorzugt nur drei Teile, sind wesentlich für dieses Zählrad. Insbesondere ist von Bedeutung, dass das Halterungsteil 3 mit zusätzlichen Funktionen und Funktionsteilen zugleich, bevorzugt einteilig materialeinheitlich, ausgebildet ist.

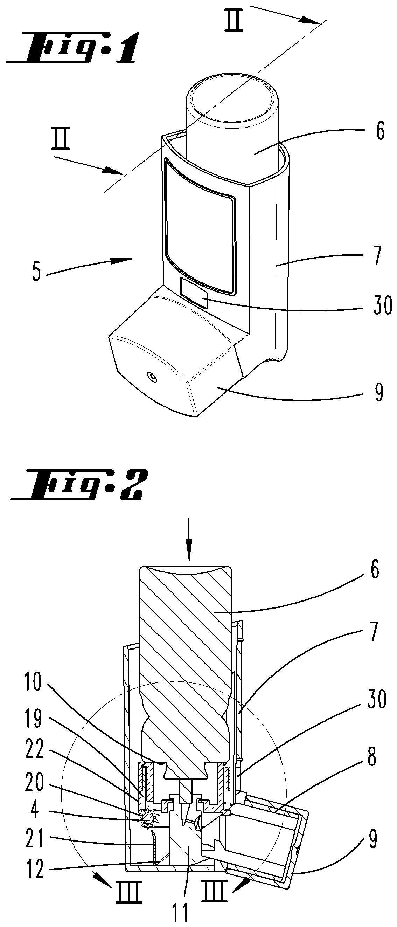

Das Zählwerk 1 kann in einem Handgerät 5, wie es grundsätzlich in Figur 1 wiedergegeben ist, angeordnet sein.

Wie sich weiter aus der Querschnittsdarstellung der Figur 2 ergibt, kann in dem Handgerät 5 ein Vorratsbehältnis 6 angeordnet sein, in dem auszugebende pharmazeutische Substanz enthalten ist. Insbesondere handelt es sich bei dem Handgerät 5 um eine Inhaliervorrichtung.

Das Handgerät 5 weist weiter ein Gehäuse 7 auf, mit einem im Benutzungszustand vertikalen Teil, in welchem ein Vorratsbehältnis 6 aufgenommen ist und einem hiervon winklig, bspw. recht- oder stumpfwinklig, bezogen auf eine Längsachse des vertikalen Teils und einer Seitenansicht, abragenden Mundstück 8. Das Mundstück 8 kann im nicht benutzten Zustand von einer Verschlusskappe 9 überfangen sein.

Das Zählwerk 1 ist insgesamt in das Gehäuse 7 des Handgerätes 5 aufgenommen.

Das Zählwerk 1 kann, wie in Figur 2 auch angedeutet, insbesondere unterseitig des im Längsquerschnitt stufenartig unterseitig gebildeten Vorratsbehältnis 6 angeordnet sein, und zwar derart, dass es mit dem Halterungsteil 3 unmittelbar in Anlage kommen kann zu einer Stufenfläche, und zwar bevorzugt einer äußeren Stufenfläche 10 des Vorratsbehältnisses 6. Das Vorratsbehältnis 6 wird zur Betätigung gegen eine in diesem selbst ausgebildete, hier im Einzelnen nicht dargestellte Feder niedergedrückt, und zwar relativ zu einem feststehenden Aufnahme- und Ausgabestumpf 11 in dem Gehäuse 7.

Hierbei wird entsprechend das Halterungsteil 3 zusammen mit dem Zählrad 2 gegen eine bevorzugt an dem Halterungsteil auch ausgebildete Rückstellfeder 12 niedergedrückt. Bei diesem Niederdrücken wirkt das Antriebsteil 4 mit dem Zählrad 1 zusammen, um dieses in Bezug auf eine Mittelachse A des Zählrades 1, welche auch eine Zylinderachse sein kann, wie nachstehend noch angesprochen, drehend anzutreiben.

Das beschriebene Zählwerk 1 ist vornehmlich nicht zu einer Einzelzählung bei jeder Betätigung des Handgerätes 5 vorgesehen. Es kann allerdings auch ein Einzelzählwerk sein. Bevorzugt zeigt das Zählwerk nur nach einer bestimmten Anzahl von Betätigungen eine neue Ziffer (vollständig) an, beispielsweise nach 5, 10 oder 20 jeweiligen Betätigungen.

Das Zählrad 2 weist entsprechende lesbare Zeichen 13, wie dargestellt auf einer Umfangsaußenfläche der bevorzugt zylindrischen Gestaltung des Zählrades 2, auf.

Die Zeichen 13 sind durch ein Fenster 30 im Gehäuse 7 lesbar.

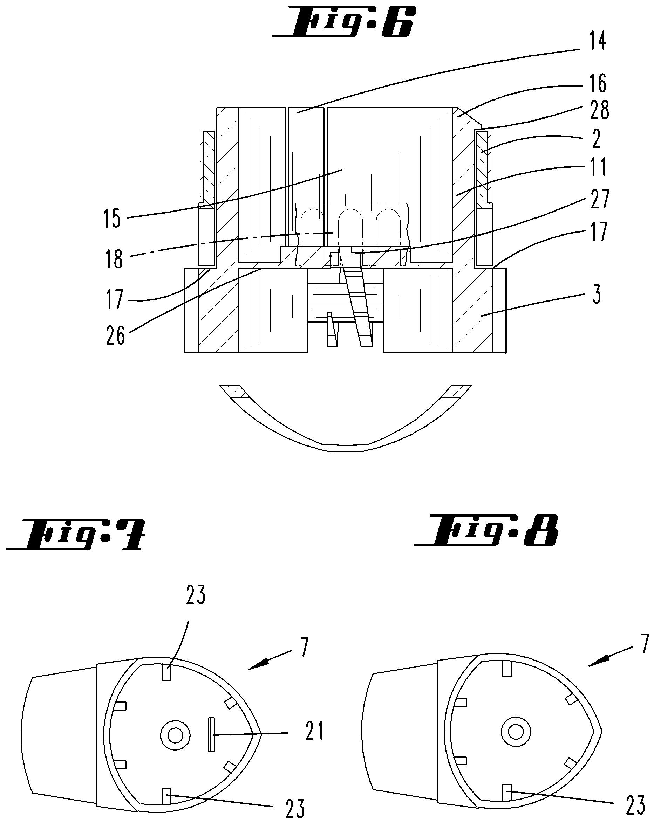

Das Zählrad 2 ist an dem Halterungsteil 3 gehaltert. Wie im Einzelnen etwa aus Figur 6 ersichtlich, hintergreift das Halterungsteil 3 hierzu das Zählrad 2 mit Halterungsfingern 14 und/oder einer Halterungswand 15. Die Halterungsfinger 14 können elastisch ausbiegbar vorgesehen sein. Aufgrund einer Einführschräge 16 an dem Halterungsfinger 14 kann das Zählrad 2 zur Befestigung an dem Halterungsteil 3 einfach von oben koaxial auf das Halterungsteil 3 aufgeschoben werden. Die Halterungsfinger 14 können nach innen ausweichen und bei vollständig aufgesetztem Zählrad 2 in der aus Figur 4 ersichtlichen Weise durch elastische Rückstellung mit einer oberen Übergriff-Ausformung das Zählrad 2 überfassen. Unterseitig kann das Zählrad 2 auf einer Stufenfläche 17 des Halterungsteils 3 aufsitzen. Das Halterungsteil 3 weist weiter bevorzugt einen Boden 26 auf. Der Boden 26 weist darüber hinaus bevorzugt eine Halterungsöffnung 27 auf, mit welcher das Zählwerk 1 im Einbauzustand, vgl. Fig. 2, an dem Aufnahme-/Ausgabestumpf 11 des Gehäuses 7 rastgehaltert sein kann.

Weiter bevorzugt ist der Boden 26 etwa auf der Höhe der Stufenfläche 17 ausgebildet. Der Boden ist darüber hinaus bevorzugt nur innenseitig der Halterungswand und im Wesentlichen senkrecht zu der Mittelachse A verlaufend ausgebildet.

In der Halterungsöffnung 27 können noch Rastfinger ausgebildet sein, welche eine mögliche Halterung des Zählwerks an dem Aufnahme-/Ausgabestumpf 11 des Gehäuses der Vorrichtung begünstigen können.

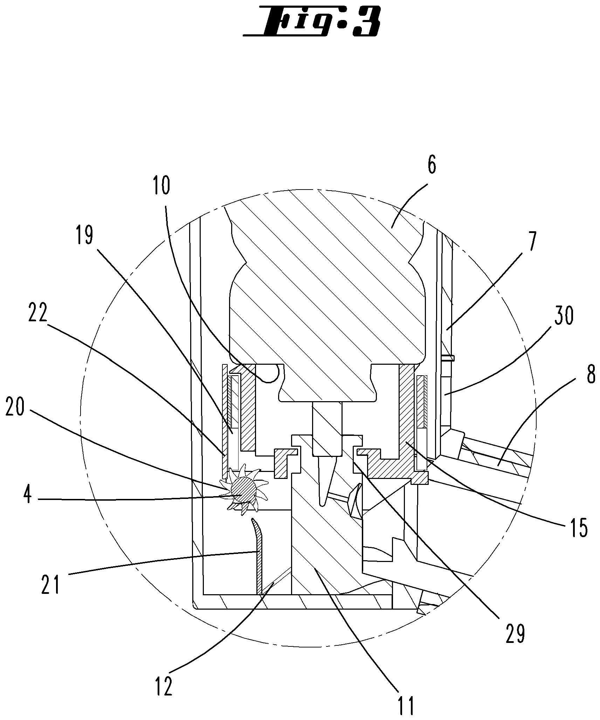

Ersichtlich weist der Halterungsstumpf 11 hierzu eine umlaufende Nut 29 auf, in der solche Rastfinger eingreifen können. In der Darstellung der Figur 3 befindet sich der Boden 26 und insbesondere auch die genannten Rastfinger in einer oberen Stellung bezüglich der Nut 29, da es sich um die unbelastete Stellung handelt und das Zählwerk 3 durch die Rückstellfeder 12 in die obere Stellung gedrückt ist. Im Zuge einer Betätigung kann der Boden und das Zählwerk 1 insgesamt relativ zu dem Gehäuse und insbesondere relativ zu dem Aufnahme-/Ausgabestumpf 11 um die vertikale Höhe (Breite) der Nut 29 sich bewegen.

Das Zählrad 2 ist unterseitig mit einer Eingriffsverzahnung 18 ausgebildet. Hierüber kann zur Drehung auf das Zählwerk 1 eingewirkt werden.

Die Einwirkung auf die Eingriffsverzahnung 18 erfolgt durch das Antriebsteil 4.

Das Halterungsteil 3 ist bevorzugt auch kreisringförmig, weiter bevorzugt zylindrisch, gegebenenfalls in einer Höhenrichtung einer Zylinderachse, die bevorzugt mit der Mittelachse A zusammenfällt, mit unterschiedlichen Bereichen ausgebildet. Unterhalb der Stufenfläche ist vorzugsweise ein Funktionsabschnitt ausgebildet. In diesem Funktionsabschnitt, der bevorzugt auch ringförmig und weiter bevorzugt zylinderförmig ist, sind bevorzugt eine oder mehrere Ausformungen vorgesehen, die eine oder mehrere Funktionen erbringen können. Zunächst eine Halterungsausformung 25 zur Halterung des Antriebsteils 4. Es kann sich um Rastöffnungen für eine Achse des Antriebsteils 4 handeln. Weiter rippenartige Gegenausformungen 24 zur Halterung in einem Gehäuse des Handgerätes. Darüber hinaus die Anbindung der Rückstellfeder 12. Diese kann von einer unteren Stirnfläche des Funktionsabschnittes ausgehend sich erstrecken. Sie schließt in ihrer Erstreckungsrichtung mit der Mittelachse A, bezogen auf eine Seitenansicht gemäß Figur 2, in der sich die Mittelachse A als Linie und die Drehachse D als Punkt darstellt, bevorzugt einen spitzen Winkel ein. Die Rückstellfeder 12 ist bevorzugt bügelartig gebildet, weiter bevorzugt derart, dass der Bügel in der genannten Seitenansicht mit seinen beiden Bügelarmen in Überdeckung ist, so dass nur der Bügelarm sichtbar ist.

Die Rückstellfeder 12 ist weiter bevorzugt hinsichtlich der bügelartigen Ausbildung geschlossen ausgebildet. Zwei Endbereiche des Bügels sind bevorzugt an unterschiedlichen Stellen unterseitig des Halterungsteils 3 im Übrigen (also für diese Zwecke gesehen ohne die Rückstellfeder 12) in dieses übergehend gebildet.

Das Antriebsteil 4 ist in weiterer Einzelheit als Schneckenwelle, bevorzugt eingängige Schneckenwelle, ausgebildet. Dies ist bspw. auch aus Fig. 6 zu ersehen. Eine Schneckenwendelsteigung kann angepasst an die Beabstandung zweier benachbarter Zahnvorsprünge 19 des Zählrades 2 gebildet sein. Hierdurch, und durch die bezüglich einer Umdrehung vorgegebene Mitnahme um einen bestimmten Betrag eines Zahnvorsprunges 19, kann die tatsächliche Zählwirkung in Bezug auf eine jeweilige Betätigung eingestellt werden.

Ein jeweiliger Mitnahmevorsprung 20, der zugleich auch als Zahn zur Einwirkung des Eingriffsteils 21 dienen kann, kann im Einzelnen zwischen zwei Mitnahmevorsprüngen 20 treten und aufgrund der wendelförmigen beziehungsweise schneckenartigen Gestaltung dann bei einer Drehung eine Mitnahme des Zählrades 2 bewirken.

Der Eingriffsteil 21 ist bevorzugt an der Rückstellfeder 12 ausgebildet, und zwar weiter bevorzugt materialeinheitlich mit der Rückstellfeder 12.

Wie darüber hinaus beispielsweise aus Figur 5 ersichtlich, kann das Eingriffsteil 21 an einem quer zu der Drehachse D am weitesten vorspringenden Bereich der Rückstellfeder 12 angebracht sein, demgegenüber aber bevorzugt wieder zurücckragend in Richtung auf die Mittelachse A.

Alternativ kann das Eingriffsteil 21, wie etwa aus Fig. 7 und, da es dort nicht an der Rückstellfeder 12 angeformt ist, aus Fig. 4 hervorgeht, unmittelbar an dem Gehäuse 7 angebracht sein. Es kann hieran steckgehaltert sein. Weiter bevorzugt kann es einteilig materialeinheitlich an das Gehäuse 7 angeformt sein.

Wie sich beispielsweise aus den Figuren 5 und 7 hervorgeht, ist das Eingriffsteil 21 auch bevorzugt schwertartig, gerade verlaufend, so dass in Fig. 7 nur der schmale rechteckige Grundriss ersichtlich ist, gebildet. Entsprechend der Ausführung gemäß Fig. 2 und Fig. 3 kann oberseitig, am freien Ende des Eingriffsteils 21 auch eine gewisse gekrümmte oder abgeknickte Ausformung vorgesehen sein.

Bei einer Betätigung des Handgerätes, also beim Ausführungsbeispiel einem Niederdrücken des Vorratsbehältnisses, verbleibt der Eingriffsvorsprung 21 etwa ortsfest relativ zu dem Gehäuse 7. Es kann sein, dass er sich im Falle der Anformung an beziehungsweise Verbindung mit der Rückstellfeder 12 zufolge der Einfederungsbewegung der Feder in geringem Ausmaß quer zu der Mittelachse A bewegt.

Bei einer Betätigung des Handgerätes trifft der Eingriffsvorsprung 21 auf einen Mitnahmevorsprung 20 des Antriebsteils 4 und bewirkt eine Drehung der Schneckenwelle um die Drehachse D des Antriebsteils 4.

Zugleich wird eine Rückdrehung des Antriebsteils 4 gehindert durch ein Sperrteil 22. Auch das Sperrteil 22 wirkt mit der Schneckenwelle, konkret den die Schneckenausbildung bildenden Zahnvorsprüngen 19 des Antriebsteils 4, zusammen.

Die Zusammenwirkung des Sperrteils 22 mit dem Antriebsteil 4 ist - bezogen etwa auf eine Seitenansicht gemäß Figur 2 oder Figur 3, in welcher sich die Drehachse D des Antriebsteils 4 punktförmig abbildet -, gegenüberliegend zu der entsprechenden Zusammenwirkung des Antriebsteils 4 mit dem Eingriffs teil 21.

Das Sperrteil 22 und das Eingriffsteil 21 sind bevorzugt entgegengesetzt gerichtet ausgebildet. Es kann sich jeweils um in Richtung der Mittelachse A und etwa parallel hierzu sich erstreckende Flachteile handeln. Das Eingriffsteil 21 und/oder das Sperrteil 22 kann oberseitig beziehungsweise zugeordnet dem jeweiligen freien Ende noch eine hakenförmige Ausbildung aufweisen, zur günstigeren Zusammenwirkung mit einem Mitnahmevorsprung 20.

Wie sich aus Figur 7 ergibt, weist das Gehäuse 7 des Handgerätes innenseitig über den Umfang verteilte Rippenausformungen 23 auf. Diese Rippenausformungen 23 dienen zur Drehsicherung des Zählwerks 1. Sie können hierzu mit den Gegenausformungen 24 an dem Halterungsteil 3 zusammenwirken. Sie können weiter auch eine Führungsbegrenzung für die Rückstellfeder 12 bilden.

**Bezugszeichenliste:**

| | | | |
|---|---|---|---|
| 1 | Zählwerk | 25 | Halterungsvorsprung |
| 2 | Zählrad | 26 | Boden |
| 3 | Halterungsteil | 27 | Halterungsöffnung |
| 4 | Antriebsteil | 28 | Übergriff-Ausformung |
| 5 | Handgerät | 29 | Nut |
| 6 | Vorratsbehältnis | 30 | Fenster |
| 7 | Gehäuse | | |
| 8 | Mundstück | | |
| 9 | Verschlusskappe | | |
| 10 | Stufenfläche | | |
| 11 | Aufnahme-/Ausgabestumpf | | |
| 12 | Rückstellfeder | | |
| 13 | Zeichen | | |
| 14 | Halterungsfinger | | |
| 15 | Halterungswand | | |
| 16 | Einführschräge | | |
| 17 | Stufenfläche | | |
| 18 | Eingriffsverzahnung | | |
| 19 | Zahnvorsprung | A | Mittelachse |
| 20 | Mitnahmevorsprung | D | Drehachse |
| 21 | Eingriffsteil | | |
| 22 | Sperrteil | | |
| 23 | Rippenausformung | | |
| 24 | Gegenausformung | | |

## Patentansprüche

1. Handgerät (5) zur Ausgabe einer pharmazeutischen Substanz, mit einem Gehäuse (7) und einem Zählwerk (1), wobei das Zählwerk (1) mindestens ein lesbare Zeichen (13) aufweisendes Zählrad (2) und ein zur Drehung des Zählrades (2) ausgebildetes Antriebsteil (4) aufweist, wobei das Zählrad (2) drehbar an einem Halterungsteil (3) angebracht ist, das Halterungsteil (3) unabhängig von dem Gehäuse (7) ausgebildet ist und das Antriebsteil (4) unmittelbar an dem Halterungsteil (3) angebracht ist, **dadurch gekennzeichnet, dass** das Zählrad (2) zylindrisch ausgebildet ist und mit einer an einer im Benutzungszustand unteren Randkante ausgebildeten Eingriffsverzahnung (18) versehen ist, auf welche das Antriebsteil (4) zur Drehung auf das Zählrad (2) einwirkt, wobei eine nach oben verbleibende Außenfläche des Zählrades (2) zur Anbringung von lesbaren Zeichen frei nutzbar ist, und dass zur Durchführung einer Zählung das Halterungsteil (3) zusammen mit dem Antriebsteil (4) zu dem relativ zu dem Gehäuse (7) praktisch feststehenden, allenfalls etwa im Sinne einer Bewegung quer zur Einfederungsbewegung bei einer Betätigung bewegten Eingriffsteil (21) bewegt ist.

2. Zählwerk (1) für ein Handgerät (5) zur Ausgabe einer pharmazeutischen Substanz mit mindestens einem lesbare Zeichen (13) aufweisenden Zählrad (2) und einem zur Drehung des Zählrades (2) ausgebildeten Antriebsteil (4) mit einer Drehachse (D), wobei das Zählwerk (1) eine Rückstellfeder (12) zur Rückverlagerung nach einer Beaufschlagung aufweist und die gesondert von dem Antriebsteil (4) ausgebildete Rückstellfeder (12) ein Eingriffsteil (21) zur Zusammenwirkung mit dem Antriebsteil (4) aufweist, wobei darüber hinaus das Zählrad (2) drehbar an einem Halterungsteil (3) angebracht ist, **dadurch gekennzeichnet, dass** die in einer Beaufschlagungsrichtung unterhalb des Halterungsteils (3) angeordnete Rückstellfeder (12) in Form eines Bügels gebildet ist, der zwei Endbereiche aufweist, die an dem Halterungsteil (3) befestigt sind, wobei die Endbereiche an unterschiedlichen Stellen unterseitig des Halterungsteils (3) in dieses übergehen und dass zur Durchführung einer Zählung das Halterungsteil (3) zusammen mit dem Antriebsteil (4) relativ zu dem Eingriffsteil (21) bewegbar ist.

3. Handgerät oder Zählwerk nach einem der vorhergehenden Ansprüche, **dadurch gekennzeichnet, dass** die Drehachse (D) außenseitig des Zählrades (29) verläuft, auch im Hinblick auf eine Projektion des Zählrades (2) in Richtung einer Mittelachse (A) des Zählrades (2).

4. Handgerät oder Zählwerk nach einem der vorhergehenden Ansprüche, **dadurch gekennzeichnet, dass** das Eingriffsteil (21) an dem Halterungsteil (3) vermittels der Rückstellfeder (12) angebracht ist.

5. Handgerät oder Zählwerk nach einem der vorhergehenden Ansprüche, **dadurch gekennzeichnet, dass** an dem Halterungsteil (3) ein Sperrteil (22) zur Einwirkung auf das Antriebsteil (4) angebracht ist.

6. Handgerät oder Zählwerk nach Anspruch 5, **dadurch gekennzeichnet, dass** das Sperrteil (22) und das Eingriffsteil (21) gegensinnig gerichtet angeordnet sind.

7. Handgerät oder Zählwerk nach einem der vorhergehenden Ansprüche, **dadurch gekennzeichnet, dass** die Drehachse (D) quer zu einer Mittelachse (A) des Zählrades (2) verläuft.

8. Handgerät oder Zählwerk nach einem der vorhergehenden Ansprüche, **dadurch gekennzeichnet, dass** das Eingriffsteil (21) zählradseitig der Drehachse (D) auf das Antriebsteil (4) einwirkt.

9. Handgerät oder Zählwerk nach einem der Ansprüche 1 bis 7, **dadurch gekennzeichnet, dass** das Sperrteil (22) auf einer dem Zählrad (2) abgewandten Seite der Drehachse (D) des Antriebsteils (4) auf das Antriebsteil (4) einwirkt.

10. Handgerät oder Zählwerk nach einem der vorhergehenden Ansprüche, **dadurch gekennzeichnet, dass** das Antriebsteil (4) als Schneckenwelle mit Verzahnungsvorsprüngen als Umfangsverzahnung ausgebildet ist, mit einem zu einer Drehachse (D) des Antriebsteils (4) schräg verlaufenden, sich schneckenartig um die Drehachse (D) erstreckenden Wendelgang.

## Claims

1. A handheld device (5) for dispensing a pharmaceutical substance with a housing (7) and a counter (1), wherein the counter (1) features at least one counter wheel (2) with readable characters (13) and a drive part (4) designed for rotating the counter wheel (2), wherein the counter wheel (2) is rotatably attached to a mounting part (3), wherein the mounting part (3) is realized independently of the housing (7), and wherein the drive part (4) is directly attached to the mounting part (3), **characterized in that** the counter wheel (2) is realized cylindrically and provided with an engagement toothing (18), which is realized on a lower peripheral edge during the intended use and acted upon by the drive part (4) in order to realize the rotation of the counter wheel (2), wherein a remaining outer surface of the counter wheel (2) toward the top can be freely used for applying readable characters, and **in that** the mounting part (3) and the drive part (4) are jointly moved relative to the engagement part (21), which practically is stationary with respect to the housing (7) and at best slightly moved in the form of a motion transverse to the deflecting motion during an actuation, in order to carry out a counting process.

2. A counter (1) for a handheld device (5) for dispensing a pharmaceutical substance with at least one counter wheel (2) with readable characters (13) and a drive part (4) with a rotational axis (D) designed for rotating the counter wheel (2), wherein the counter (1) features a return spring (12) for the retrodisplacement after an actuation, wherein the spring (12), which is realized separately of the drive part (4), features an engagement part (21) for interacting with the drive part (4), and wherein the counter wheel (2) is furthermore rotatably attached to the mounting part (3), **characterized in that** that the return spring (12), which is arranged underneath the mounting part (3) referred to an actuating direction, is realized in the form of a bow with two end regions that are fastened on the mounting part (3), wherein the end regions transform into the mounting part (3) at different locations on the underside thereof, and **in that** the mounting part (3) and the drive part (4) can be jointly moved relative to the engagement part (21) in order to carry out a counting process.

3. The handheld device or counter according to one of the preceding claims, **characterized in that** the rotational axis (D) extends outside the counter wheel (29), namely also with respect to a projection of the counter wheel (2) in the direction of a central axis (A) of the counter wheel (2).

4. The handheld device or counter according to one of the preceding claims, **characterized in that** the engagement part (21) is attached to the mounting part (3) by means of a return spring (12).

5. The handheld device or counter according to one of the preceding claims, **characterized in that** a locking part (22) for acting upon the drive part (4) is attached to the mounting part (3).

6. The handheld device or counter according to claim 5, **characterized in that** the locking part (22) and the engagement part (21) are arranged such that they are directed oppositely.

7. The handheld device or counter according to one of the preceding claims, **characterized in that** the rotational axis (D) extends transverse to a central axis (A) of the counter wheel (2).

8. The handheld device or counter according to one of the preceding claims, **characterized in that** the engagement part (21) acts upon the drive part (4) on the counter wheel side of the rotational axis (D).

9. The handheld device or counter according to one of claims 1-7, **characterized in that** the locking part (22) acts upon the drive part (4) on the side of the rotational axis (D) of the drive part (4), which faces away from the counter wheel (2).

10. The handheld device or counter according to one of the preceding claims, **characterized in that** the drive part (4) is realized in the form of a worm shaft with toothing projections that form a circumferential toothing with a helix, which extends obliquely to a rotational axis (D) of the drive part (4) and helically around the rotational axis (D).

## Revendications

1. Appareil à main (5) pour la distribution d'une substance pharmaceutique, comportant un boîtier (7) et un mécanisme de comptage (1), dans lequel le mécanisme de comptage (1) comprend au moins une roue de comptage (2) présentant des caractères lisibles (13) et un élément d'entraînement (4) conçu pour faire tourner la roue de comptage (2), dans lequel la roue de comptage (2) est montée à rotation sur une pièce de support (3), la pièce de support (3) est formée indépendamment du boîtier (7) et l'élément d'entraînement (4) est monté directement sur la pièce de support (3), **caractérisé en ce que** la roue de comptage (2) est cylindrique et est pourvue d'une denture d'engagement (18) qui est formée sur un bord inférieur dans l'état d'utilisation et sur laquelle agit l'élément d'entraînement (4) pour faire tourner la roue de comptage (2), dans lequel une surface extérieure de la roue de comptage (2) qui subsiste vers le haut est librement utilisable pour l'apposition de caractères lisibles, et **en ce que**, pour effectuer un comptage, la pièce de support (3) ensemble avec l'élément d'entraînement (4) est déplacé vers l'élément d'engagement (21) qui pratiquement est fixe par rapport au boîtier (7) et qui tout au plus au sens d'un mouvement est quelque peu déplacé transversalement au mouvement de déflexion lors d'un actionnement.

2. Mécanisme de comptage (1) pour un appareil à main (5) pour la distribution d'une substance pharmaceutique, comprenant au moins une roue de comptage (2) présentant des caractères lisibles (13) et un élément d'entraînement (4) ayant un axe de rotation (D), qui est conçu pour faire tourner la roue de comptage (2), dans lequel le mécanisme de comptage (1) comprend un ressort de rappel (12) pour le déplacement en retour après une sollicitation et le ressort de rappel (12) formé séparément de l'élément d'entraînement (4) présente un élément d'engagement (21) pour coopérer avec l'élément d'entraînement (4), dans lequel, en outre, la roue de comptage (2) est jointe de manière tournante à une pièce de support (3), **caractérisé en ce que** le ressort de rappel (12), agencé sous la pièce de support (3) dans la direction de sollicitation, est réalisé sous la forme d'un étrier présentant deux zones d'extrémité qui sont fixées à la pièce de support (3), les zones d'extrémité se raccordant à la pièce de support (3) en différents points sur le côté inférieur de celle-ci, et **en ce que** pour effectuer un comptage, la pièce de support (3) ensemble avec l'élément d'entraînement (4) est mobile par rapport à l'élément d'engagement (21).

3. Appareil à main ou mécanisme de comptage selon l'une des revendications précédentes, **caractérisé en ce que** l'axe de rotation (D) s'étend à l'extérieur de la roue de comptage (29), également en ce qui concerne une projection de la roue de comptage (2) dans la direction d'un axe central (A) de la roue de comptage (2).

4. Appareil à main ou mécanisme de comptage selon l'une des revendications précédentes, **caractérisé en ce que** l'élément d'engagement (21) est joint à la pièce de support (3) au moyen du ressort de rappel (12).

5. Appareil à main ou mécanisme de comptage selon l'une des revendications précédentes, **caractérisé en ce qu'**un élément de blocage (22) pour agir sur l'élément d'entraînement (4) est joint à la pièce de support (3).

6. Appareil à main ou mécanisme de comptage selon la revendication 5, **caractérisé en ce que** l'élément de blocage (22) et l'élément d'engagement (21) sont agencés avec une orientation en opposition.

7. Appareil à main ou mécanisme de comptage selon l'une des revendications précédentes, **caractérisé en ce que** l'axe de rotation (D) s'étend transversalement à un axe central (A) de la roue de comptage (2).

8. Appareil à main ou mécanisme de comptage selon l'une des revendications précédentes, **caractérisé en ce que** l'élément d'engagement (21) agit sur l'élément d'entraînement (4) du côté de la roue de comptage de l'axe de rotation (D).

9. Appareil à main ou mécanisme de comptage selon l'une des revendications 1 à 7, **caractérisé en ce que** l'élément de blocage (22) agit sur l'élément d'entraînement (4) sur un côté de l'axe de rotation (D) de l'élément d'entraînement (4) qui est éloigné de la roue de comptage (2).

10. Appareil à main ou mécanisme de comptage selon l'une des revendications précédentes, **caractérisé en ce que** l'élément d'entraînement (4) est réalisé sous la forme d'un arbre à vis sans fin avec des saillies de denture en tant que denture périphérique, avec une hélice s'étendant obliquement par rapport à un axe de rotation (D) de l'élément d'entraînement (4) et de manière hélicoïdale autour de l'axe de rotation (D).
